**Europäisches Patentamt**

**European Patent Office**

**Office européen des brevets**

(19)

(11) **EP 1 279 368 A2**

(12) **EUROPÄISCHE PATENTANMELDUNG**

(43) Veröffentlichungstag:
**29.01.2003 Patentblatt 2003/05**

(51) Int Cl.[7]: **A61B 5/087**, G01F 1/66

(21) Anmeldenummer: **02015706.1**

(22) Anmeldetag: **12.07.2002**

(84) Benannte Vertragsstaaten:
**AT BE BG CH CY CZ DE DK EE ES FI FR GB GR
IE IT LI LU MC NL PT SE SK TR**
Benannte Erstreckungsstaaten:
**AL LT LV MK RO SI**

(30) Priorität: **17.07.2001 DE 10134746
20.11.2001 DE 10156854**

(71) Anmelder: **ndd Medizintechnik AG
8005 Zürich (CH)**

(72) Erfinder:
• **Buess, Christian, Dr.
8008 Zürich (CH)**
• **Kleinhappl, Erich
8820 Wädenswil (CH)**

(74) Vertreter: **Laufhütte, Dieter, Dr.-Ing. et al
Lorenz-Seidler-Gossel
Widenmayerstrasse 23
80538 München (DE)**

(54) **Vorrichtung zur Messung der Strömungsgeschwindigkeit und/oder der Molmasse von Gasen- oder Gasgemischen**

(57) Die Erfindung betrifft eine Vorrichtung zur Messung der Strömungsgeschwindigkeit und/oder der Molmasse von Gasen-oder Gasgemischen in medizinischer Anwendung mittels Ultraschall-Laufzeitmessung mit einem Meßrohr und zwei in dieses einsetzbaren Ultraschallwandlern, die über Membranen vom Meßrohrinnenraum getrennt sind. Erfindungsgemäß sind die Membranen gasdicht ausgeführt und so in das Meßrohr eingesetzt, daß dieses eine gasdichte Rohrverbindung bildet. Die wieder trennbar mit dem Meßrohr verbundenen Ultraschallwandler kontaktieren die Membran bündig.

Figur 2a

**(Forts. nächste Seite)**

EP 1 279 368 A2

Figur 2b

**Beschreibung**

Die Erfindung betrifft eine Vorrichtung zur Messung der Strömungsgeschwindigkeit und/oder der Molmasse von Gasen- oder Gasgemischen in medizinischer Anwendung mittels Ultraschall-Laufzeitmessung.

Aus der DE 42 22 286 C ist bereits ein sogenanntes Ultraschall-Spirometer bekannt, bei dem zur Vermeidung von Kreuzinfektionen die Verwendung eines leicht austauschbaren weitgehend sterilen Atemrohres empfohlen wird, das in das Meßrohr einführbar ist. Dieses Atemrohr weist am Übergang zu der Meßstrecke Meßfenster in der Art auf, daß in entsprechenden Öffnungen Einsätze eingesetzt sind, die durchlässig für Ultraschallwellen, aber weitgehend undurchlässig für Keime und Verschmutzungen sind. Bei dieser Lösung sind die jeweils das Sende- bzw. Empfangszellenpaar bildenden Ultraschallwandler in einer Meßstrecke schräg zur Meßrohrachse angeordnet. Hierdurch bedingt ist es, daß seitlich an dem Meßrohr Kammern angeformt sind, in welchen die jeweiligen Ultraschallwandler sitzen. Diese seitlich angebrachten Kammern führen aber dazu, daß bei der Bestimmung der Molmasse nicht nur das in der Meßstrecke befindliche Gas bestimmt wird sondern auch das in den Kammern befindliche Gas bzw. Gasgemisch.

Aufgabe der Erfindung ist es, eine gattungsgemäße Vorrichtung zur Messung der Strömungsgeschwindigkeit und/oder der Molmasse von Gasen- oder Gasgemischen in medizinischer Anwendung mittels Ultraschall-Laufzeitmessung derart weiter zu bilden, daß bei der Ultraschallmessung ausschließlich die Molmasse des in der Meßstrecke befindlichen Gases bestimmbar ist, wobei eine vollständige Sterilisierbarkeit der Meßstücke erzielt wird.

Diese Aufgabe wird erfindungsgemäß durch die Merkmalskombination des Anspruchs 1 gelöst. Gemäß dieser Lösung werden ausgehend von der gattungsgemäßen Vorrichtung die Membranen gasdicht ausgebildet, und so in das Meßrohr eingesetzt, daß dieses eine gasdichte Rohrverbindung bildet. Die Ultraschallwandler kontaktieren bündig die jeweiligen Membranen.

Die Hauptanwendung dieser Lösung liegt im Einsatz einer entsprechenden Vorrichtung zur Messung der Atemströmungsgeschwindigkeit und der Atemgaszusammensetzung bei normal atmenden oder maschinell beatmeten Patienten. In dieser Anwendung kann die erfindungsgemäße Vorrichtung in den Atemstrom bzw. Atemkreislauf eingefügt werden. Aufgrund der gemessenen Parameter (Strömungsgeschwindigkeit, Molmasse, Druck) können diverse für die Lungenfunktionsdiagnostik wichtige Parameter bestimmt werden. Beispiele hierfür sind Atemzugsvolumina, die funktionelle Residualkapazität, welche mittels Auswasch- oder Einwaschmethoden und einer Analyse von Strömungsgeschwindigkeit und Molmasse bestimmbar ist, sowie Diagramme, welche den Zusammenhang zwischen Druck, Strömungsgeschwindigkeit, Molmasse oder Volumen darstellen.

Bevorzugte Ausführungsformen der Erfindung ergeben sich aus den sich an den Hauptanspruch anschließenden Unteransprüchen.

Die Membranen können derart angeordnet sein, daß die Ultraschallmeßstrecke parallel zur Rohrsymmetrieachse liegt.

Gemäß einer vorteilhaften Ausgestaltung können die Anschlüsse zur Zuführung des Gases seitlich an das Meßrohr, d.h. senkrecht zur Meßrohrachse, angesetzt sein.

Es sind vorzugsweise elektronische Mittel vorhanden, mit deren Hilfe aus den Schallaufzeiten der über die Membran in das Meßrohr eingekoppelten Ultraschallimpulse die Strömungsgeschwindigkeit und/oder die Molmasse berechnet werden.

Zusätzlich können ein oder mehrere Meßfühler zur Bestimmung der Gas- und/oder Gehäusetemperatur vorhanden sein.

Die Strömung wird vorzugsweise nach folgender an sich schon bekannten Formel berechnet:

$$F = k\,\frac{t_1 - t_2}{t_1 \cdot t_2}$$

wobei F die Strömungsgeschwindigkeit, $t_1$ und $t_2$ die mittels Ultraschall-Laufzeitmessung gemessenen Laufzeiten der Ultraschallimpulse und k eine dimensionsbehaftete Konstante bezeichnen.

Vorzugsweise wird die Molmasse der Gase- oder Gasgemische nach der auch bereits bekannten Formel berechnet:

$$M = k \cdot T \cdot \left(\frac{t_1 \cdot t_2}{t_1 + t_2}\right)^2$$

wobei M die Molmasse, T die mittels Annahmen und/oder mathematischem Modell und/oder Messung mittels einem oder mehreren in der Vorrichtung vorhanden Sensoren bestimmte Temperatur des Gases, k eine dimensionsbehaftete Konstante und $t_1$ und $t_2$ die mittels elektronischen Schaltungen gemessene Laufzeiten der Ultraschallimpulse bezeichnen.

Die Ultraschallwandler können gemeinsam oder einzeln mit dem Meßrohr verbunden sein, wobei die Ultraschallwandler bevorzugt einzeln oder als Paar mittels mechanischer Spannvorrichtung an die Membranen aufpressbar sind. Das Meßrohr ist also von den Ultraschallwandlern trennbar und ingesamt zur Reinigung oder Desinfektion bzw. bei der Einmalverwendung zur Entsorgung leicht austauschbar.

Die Membranen können aus Kunststoff oder Metall gefertigt sein.

Die vorzugsweise piezokeramischen Ultraschallwandler können zusätzlich mit einer Impedanzanpassungsschicht versehen sein.

Zwischen der Impedanzanpassungsschicht und der Membran kann zusätzlich eine gelartige Überbrückungssubstanz, ein sogenanntes "Ultraschallübertragungsgel", eingebracht sein.

Die Güte der Schallübertragung durch die Membranen kann mittels einer Empfangs-Signalamplitudenmessung überwacht bzw. geregelt sein.

Zu Berechnung zusätzlicher Parameter kann der Gasdruck im Meßrohr mittels eines geeigneten Drucksensors gemessen werden. Diese Drucküberwachung ist insbesondere bei der Verwendung der Vorrichtung in der Intensivmedizin wünschenswert.

Besonders vorteilhaft kann der Ultraschallwandler bzw. dessen Impedanzanpassungsschicht eine gewölbte Oberfläche aufweisen. Durch diese gewölbte Oberfläche wird eine besonders gute Verbindung zwischen dem Ultraschallwandler einerseits und der Membran andererseits möglich. Insbesondere werden Lufteinschlüsse verhindert, da beim Ankoppeln des Ultraschallwandlers die zwischen der Oberfläche des Ultraschallwandlers und der Membran befindliche Luft nach außen zum Rand hin weggedrängt wird und sich somit nicht in der Mitte des Ultraschallwandlers sammeln kann.

Gemäß einer weiteren besonderen Ausgestaltung der Erfindung sind die Membranen nicht unmittelbar in den Grundkörper des Meßrohres eingesetzt, sondern über eine aus einem Dämpfungsring bestehende Aufhängung. Diese aus dem Dämpfungsring bestehende Aufhängung dient zur Unterdrückung von Körperschallsignalen, d.h. von Ultraschallsignalen, welche nicht über die Luft, sondern über den Grundkörper von Wandler zu Wandler übertragen werden.

Weitere Einzelheiten und Vorteile der Erfindung werden anhand eines in der Zeichnung dargestellten Ausführungsbeispieles näher erläutert. Es zeigen

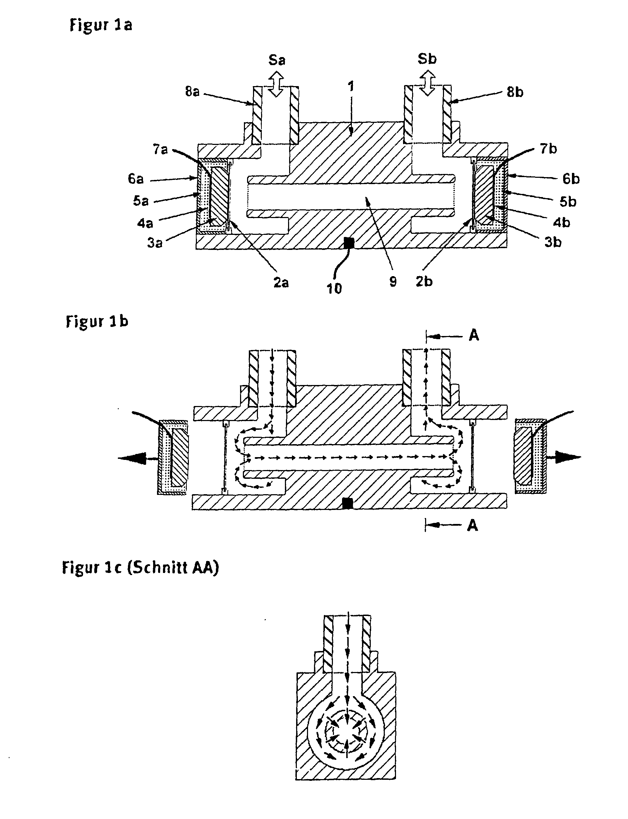

Figur 1a: eine Schnittdarstellung durch eine erste Ausführungsvariante der erfindungsgemäßen Vorrichtung,

Figur 1b: eine der Figur 1 a entsprechende Schnittdarstellung, in welcher die Ultraschallwandler von dem Meßrohr getrennt sind und in welcher der Gasstrom eingezeichnet ist,

Figur 1 c: einen Schnitt entlang der Schnittlinie A-A durch Figur 1b,

Figur 2a: eine Schnittdarstellung durch eine zweite Ausführungsvariante der erfindungsgemäßen Vorrichtung,

Figur 2b: eine der Figur 2a entsprechende Schnittdarstellung, in welcher die Ultraschallwandler vom Meßrohr getrennt sind und in welcher die Gasströmung durch Pfeile eingezeichnet ist und

Figur 3: eine Schnittdarstellung durch ein Detail einer weiteren Ausführungsvariante der erfindungsgemäßen Vorrichtung.

In den Figuren 1a, 1b, 1c ist eine Realisierungsvariante der Vorrichtung mit einzeln aufsteckbaren Ultraschallwandlern dargestellt. Das eigentliche Meßrohr besteht aus einem Grundkörper 1 mit eingelassenen Membranen 2a und 2b, die aus Kunststoff oder Metall gefertigt sind. Diese Membranen sind gasdicht ausgeführt. Dadurch kann auch ohne eingesetzte Ultraschallwandler 3a, 3b bis 7a, 7b dieses Atemrohr dicht über Anschlüsse 8a und 8b mit dem Atemkreislauf verbunden werden. Die Anschlüsse 8a und 8b sind jeweils senkrecht zur Symmetrieachse des Meßrohres angeordnet, so daß das Meßrohr nahezu eine U-Form aufweist. Im hier vorliegenden Fall strömt Luft Sa bzw. Sb über einen Anschluß, beispielsweise den Anschluß 8a in die Meßvorrichtung, wird in einen eigentlichen Meßkanal 9 geleitet und strömt über den gegenüberliegenden Anschluß, beispielsweise den Anschluß 8b, wieder aus dem Meßsystem heraus.

Das Meßrohr 1, 2a bzw. 2b beinhaltet ausschließlich mechanische Teile und kann somit ohne Probleme gereinigt oder desinfiziert werden. Alternativ kann das Meßrohr aber auch als Wegwerfteil ausgebildet sein und somit für den Einmalgebrauch dienen. Das bedeutet, daß pro Patient jeweils ein neues Meßrohr verwendet wird.

Zur Bestimmung der Strömungsgeschwindigkeit und/oder der Molmasse, der Gase im Meßkanal 9 werden zwei Ultraschallwandler auf den Grundkörper 1 gesteckt. Die Ultraschallwandler bestehen aus einer Impedanzanpassungsschicht 3a und 3b, einer Piezokeramik 4a und 4b zur Erzeugung des Ultraschalls, einer - unter Umständen mehrstufigen - Dämpfungsschicht 5a und 5b, einer Halterung 6a und 6b und Anschlußdrähten 7a und 7b. Beim Einstecken der Ultraschallwandler wird die Impedanzanpassungsschicht direkt mit der Membran 2 a und 2b in Verbindung gebracht.

Zur Bestimmung der Schallaufzeiten wird einer der beiden Ultraschallwandler mit einer Impulswellenform angeregt. Die mittels der Piezokeramik 4a erzeugte Schallwelle wird über die Impedanzanpassungsschicht 3a und die Membran 2a in den Meßkanal übertragen und wird nach Durchlaufen der Meßstrecke vom gegenüberliegenden Ultraschallwandler empfangen. Mittels einer nachgeschalteten Elektronik, die hier nicht näher dargestellt ist, wird die Laufzeit $t_1$ der Ultraschallwelle bestimmt. Kurze Zeit nach der Schallübertragung von Wandlerseite a auf Wandlerseite b wird die Übertragungsrichtung geändert und die Schallaufzeit $t_2$ von der Wandlerseite b auf die Wandlerseite a kann bestimmt werden. Die Strömungsgeschwindigkeit kann nun mittels bekannter Verfahren aus den beiden Schal-

laufzeiten $t_1$ und $t_2$ ermittelt werden.

Wird zusätzlich zur Messung der Schallaufzeiten die Temperatur des Atemgases im Meßkanal bestimmt, so kann ebenfalls die Molmasse der Gase berechnet werden. Die Bestimmung der Temperatur kann mittels Messung, mittels Annahme oder mittels geeigneter mathematischer Modelle oder mittels Kombinationen dieser Methoden bestimmt werden. Im Grundkörper 1 können zu diesem Zweck eine oder mehrere Temperatursensoren 10 vorhanden sein.

In Fig. 1b ist dargestellt, daß die Ultraschallwandler 3a, 3b bis 7a, 7b von dem Grundkörper 1 durch entsprechendes Herausziehen in Pfeilrichtung trennbar ist. Durch die Pfeile in den Figuren 1b und 1c ist die Strömungsrichtung des Gases bzw. des Gasgemisches gezeigt.

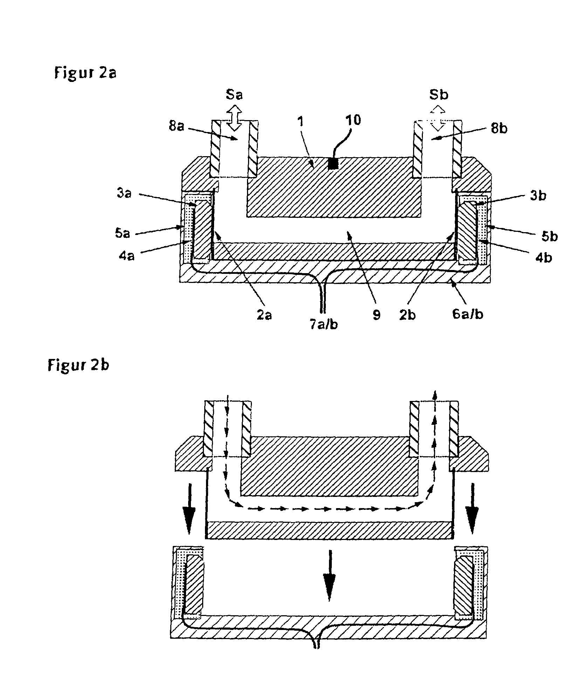

In den Figuren 2a und 2b ist eine Realisierungsvariante der erfindungsgemäßen Vorrichtung mit gemeinsam auswechselbaren Ultraschallwandlern dargestellt. Die beiden Ultraschallwandler werden in dieser Realisierungsvariante direkt über die Membranen geschoben. Letztere sind auf den Grundkörper 1 des Meßrohres beispielsweise mittels Schweißung befestigt. Gegenüber der Vorrichtung in Figur 1b wird die Luftströmung in anderer Weise in den Meßkanal geleitet.

Es sind verschiedene weitere Ausführungsvarianten der Vorrichtung möglich. So kann beispielsweise die in den Figuren 1a bis 1c dargestellte Ausführungsvariante auch mit gemeinsam auswechselbaren Ultraschallwandlern hergestellt werden. In diesem Fall werden die Wandler mit einer zangenartigen Vorrichtung aufgespannt, wie dies in der auseinandergezogenen Darstellung gemäß Fig. 2b gezeigt ist. Hier wird deutlich, daß die zangenartige Vorrichtung mit den Ultraschallwandlern in Pfeilrichtung vom Grundkörper 1 abgezogen und gegen diese Pfeilrichtung wieder mit diesem verbunden werden kann.

In hier nicht näher dargestellter Art und Weise sind die Ultraschallwandler oder entsprechend auf diesen vorgesehenen Impedanzanpassungsschichten gewölbt ausgebildet, so daß sich die gasdichten Membranen 2a, 2b dicht an deren Oberfläche anschmiegen. Hierdurch wird eine verbesserte Kopplung zwischen den Ultraschallwandlern und den gasdichten Membranen erzielt.

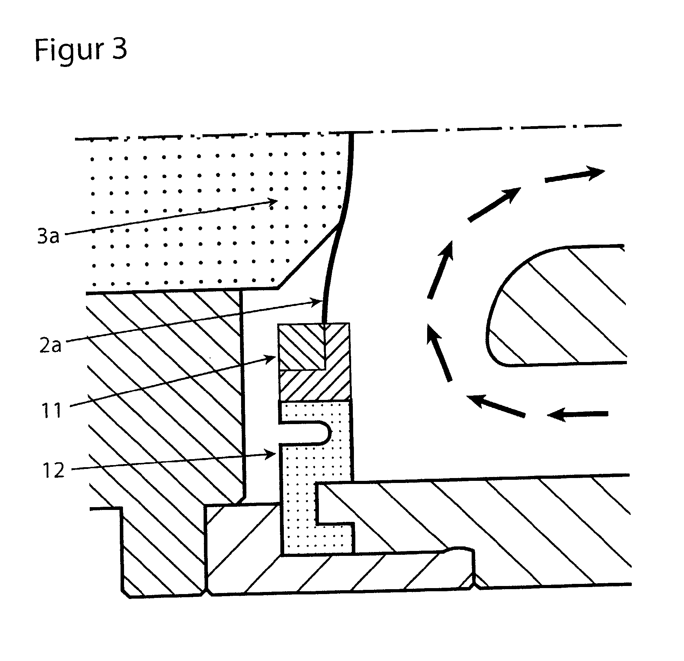

In der Schnittdarstellung gemäß Figur 3 ist ein Detail dargestellt, aus dem die schwingende Aufhängung der gasdichten Membran 2a gezeigt ist. Diese ist über eine Halterung 11 mit einem Dämpfungsring 12 verbunden, wobei der Dämpfungsring im Grundkörper des Meßrohres 1 verankert ist. Wie aus der Figur 3 ersichtlich liegt der Ultraschallwandler 3a an der Oberfläche der Membran 2a an. Um die schwingende Aufhängung zu ermöglichen, ist der Dämpfungsring aus einem flexiblen Material gefertigt, wobei er zusätzlich durch seine Formgebung die elastisch federnde Eigenschaft unterstützt. Durch die schwingende Aufhängung der Membran 2a wird verhindert, daß Ultraschallsignale über den Grundkörper des Meßrohres von Wandler zu Wandler übertragen werden. Die die Messung störenden Körperschallsignale werden also vermieden.

**Patentansprüche**

1. Vorrichtung zur Messung der Strömungsgeschwindigkeit und/oder der Molmasse von Gasen oder Gasgemischen in medizinischer Anwendung mittels Ultraschall-Laufzeitmessung mit einem Meßrohr und zwei in dieses einsetzbaren Ultraschallwandlern, die über Membranen vom Meßrohrinnenraum getrennt sind,
**dadurch gekennzeichnet,**
**daß** die Membranen gasdicht sind und so in das Meßrohr eingesetzt sind, daß dieses eine gasdichte Rohrverbindung bildet, und daß die wieder trennbar mit dem Meßrohr verbundenen Ultraschallwandler die Membranen bündig kontaktieren.

2. Vorrichtung nach Anspruch 1, **dadurch gekennzeichnet, daß** die Membranen derart angeordnet sind, daß die Ultraschallmeßstrecke parallel zur Rohrsymmetrieachse liegt.

3. Vorrichtung nach Anspruch 1 oder 2, **dadurch gekennzeichnet, daß** die Anschlüsse zur Zuführung des Gases senkrecht zur Meßrohrachse angesetzt sind.

4. Vorrichtung nach einem der Ansprüche 1 bis 3, **gekennzeichnet durch** elektronische Mittel, mit deren Hilfe aus den Schall-Laufzeiten der über die Membranen in das Meßrohr eingekoppelten Ultraschallimpulse die Strömungsgeschwindigkeit und/oder Molmasse ermittelbar ist.

5. Vorrichtung nach einem der Ansprüche 1 bis 4, **dadurch gekennzeichnet, daß** zusätzlich ein oder mehrere Meßfühler zur Bestimmung der Gas-und/oder Gehäusetemperatur vorhanden sind.

6. Vorrichtung nach Anspruch 5, **dadurch gekennzeichnet, daß** die Strömungsgeschwindigkeit durch die Formel

$$F = k \; \frac{t_1 - t_2}{t_1 \cdot t_2}$$

berechnet wird, wobei F die Strömungsgeschwindigkeit, $t_1$ und $t_2$ die mittels Ultraschallaufzeitmessung gemessenen Laufzeiten der Ultraschallimpulse und k eine dimensionsbehaftete Konstante bezeichnen.

**7.** Vorrichtung nach Anspruch 5, **dadurch gekennzeichnet, daß** die Molmasse der Gase oder Gasgemische nach der Formel

$$M = k \cdot T \cdot \left( \frac{t_1 \cdot t_2}{t_1 + t_2} \right)^2$$

berechnet wird, wobei M die Molmasse, T die mittels Annahmen und/oder mathematischem Modell und/oder Messung mittels einem oder mehreren in der Vorrichtung vorhandenen Sensoren bestimmte Temperatur des Gases, k eine dimensionsbehaftete Konstante, und $t_1$ und $t_2$ die mittels elektronischen Schaltungen gemessenen Laufzeiten der Ultraschallimpulse bezeichnen.

**8.** Vorrichtung nach einem der Ansprüche 1 bis 7, **dadurch gekennzeichnet, daß** die Ultraschallwandler gemeinsam oder einzeln mit dem Meßrohr verbunden sind.

**9.** Vorrichtung nach Anspruch 8, **dadurch gekennzeichnet, daß** die Ultraschallwandler einzeln oder als Paar mittels mechanischer Spannvorrichtung auf die Membranen aufpreßbar sind.

**10.** Vorrichtung nach einem der Ansprüche 1 bis 9, **dadurch gekennzeichnet, daß** die Membranen aus Kunststoff oder Metall gefertigt sind.

**11.** Vorrichtung nach einem der Ansprüche 1 bis 10, **dadurch gekennzeichnet, daß** die Ultraschallwandler eine Impedanzanpassungsschicht aufweisen.

**12.** Vorrichtung nach Anspruch 11, **dadurch gekennzeichnet, daß** zur besseren Schallübertragung eine vorzugsweise gelartige Überbrückungssubstanz zwischen der Impedanzanpassungsschicht der Ultraschallwandler und den Membranen eingebracht ist.

**13.** Vorrichtung nach einem der Ansprüche 1 bis 12, **dadurch gekennzeichnet, daß** die Güte der Schallübertragung durch die Membranen mittels einer Empfangs-Signalamplitudenmessung überwachbar bzw. regelbar ist.

**14.** Vorrichtung nach einem der Ansprüche 1 bis 13, **dadurch gekennzeichnet, daß** mindestens ein Drucksensor zur Messung des Gasdrucks im Meßrohr vorgesehen ist.

**15.** Vorrichtung nach einem der Ansprüche 1 bis 14, **dadurch gekennzeichnet, daß** die Ultraschallwandler bzw. deren Impedanzanpassungsschicht eine gewölbte Oberfläche aufweisen.

**16.** Vorrichtung nach einem der Ansprüche 1 bis 15, **dadurch gekennzeichnet, daß** die Membranen über einen Dämpfungsring schwingend im Grundkörper des Meßrohres aufgehängt sind.

## Figur 1a

## Figur 1b

## Figur 1c (Schnitt AA)

Figur 2a

Figur 2b

Figur 3

3a

2a

11

12